# EUROPEAN PATENT APPLICATION

(11) **EP 4 137 163 A1**
(43) Date of publication of application: **22.02.2023**
(21) Application number: 22190334.7
(22) Date of filing: 14.08.2022
(51) Int. Cl.: A61L 9/20, F24F 8/00

(54) **AIR PURIFICATION UNIT FOR A VENTILATION SYSTEM**

(30) Priority: 16.08.2021 GB 202111733
(71) Applicant: Vitec Microgenix Limited, Wilmslow, Cheshire SK9 2EA (GB)
(72) Inventor: PERKINS, Scott, Wilmslow, SK9 2EA (GB)
(74) Representative: Harrison IP Limited

(57) **Abstract**

A purification unit is disclosed for deactivating microbes in an air stream of a ventilation system. The purification unit is composed of modules within each of which flowing air is treated to deactivate microbes. Each module has a tubular housing of square cross-section, the cross-sectional areas of different modules are in a ratio of 2ⁿ : 1 to one another, n being a positive integer, and the housings of the modules are mounted in contact with one another, in series and/or in parallel, to form a single purification unit of overall rectangular cross-section.

## Description

### Field of the invention

The present invention relates to an air purification unit for a ventilation system of a building or of a mobile structure. Though described below by reference to buildings, the invention is equally applicable to ventilation systems of land vehicles, ships and aircraft.

### Background

Ventilation systems are used to circulate air in buildings, or other structures. Optionally, they may be used to introduce fresh outdoor air into the building. The air may be cooled or heated and the air quality may be improved by filtration to remove particulate matter and purification or sterilization to deactivate microbes. The term "microbes" is used herein to include bacteria, archaea, fungi (yeasts and molds), algae, protozoa, and viruses.

Known purification units, which fall into a number of categories, either do not achieve the required air quality, or else do so in an energy-inefficient manner. A first category includes fibrous filters that are designed to trap particulate matter but have been adapted to capture microbes. As microbes are much smaller in size, purification units must use smaller mesh sizes, which results in greater resistance to air flow and increased pressure drop across the filter. As an example, a HEPA filter can cause a pressure drop of up to 1000 pascals.

A second known category comprises stand-alone purifiers, i.e., air purification units that are not integrated into the ventilation system. As they operate separately from the ventilation system, stand-alone units are not as effective at removing microbes from the circulated air.

A third known category of purification units, specifically designed to deal with microbes, uses ultraviolet (UV) light, which is itself capable of deactivating microbes and, in the case of light in the UV-C range, also acts to ozonize the air. UV light, however, is only effective in providing microbial deactivation once the microbes have had a sufficient exposure to the radiation, the exposure being the product of the intensity of the UV light and the time, herein termed the dwell time, during which the microbes are irradiated.

A problem encountered in known purification units is that they do not achieve the required dwell time when the airflow rate is high or when the ductwork uses large size ducts. To achieve the desired air quality in such cases often calls for installation of individually designed systems, which results in a significant increase in cost.

### Summary of the invention

With a view to mitigating this problem, the present invention provides a purification unit for deactivating microbes in an air stream of a ventilation system, wherein the purification unit is composed of modules within each of which flowing air is treated to deactivate microbes, wherein each module has a tubular housing of square cross-section, the cross-sectional areas of different modules are in a ratio of 2ⁿ : 1 to one another, n being a positive integer, and the housings of the modules are mounted in contact and in parallel with one another to form a single purification unit of overall rectangular cross-section.

The invention allows a purification unit of any desired capacity (measured in volume per second) to be constructed by appropriate selection of modules from a limited set of modules, by mounting individual modules in parallel with one another, i.e., with their sides in contact. Because different combinations of modules can be mounted with different sides in contact with one another, it is also possible to select an overall cross-section to suit different configurations of ductwork, ranging for example from a cross-section that is square, i.e. a rectangle with sides of equal length, to a rectangle that is long and narrow.

The purification unit can also accommodate high flow rates, in some embodiments, by connecting modules in series with one another, i.e. with their ends in contact, to increase the dwell time for a given flow rate (measured in meters per second). Such connection of air purification modules in series is known from CN 2460095.

In some embodiments of the invention, a flow diverter is provided at the intake end of at least some of the modules, to cause air to flow along a helical path through the module, thereby increasing the dwell time of the air within the module.

Each diverter may comprise inclined stationary surfaces or blades, each serving to deflect tangentially air entering the module axially, that is to say in a direction parallel to the axis of symmetry of the housing of the module.

As the modules are of square cross-section, each diverter may comprise a rotationally symmetrical arrangement of four surfaces or blades.

In some embodiments, the inclination of the blades may be adjustable to vary the pitch of the helical path followed by the air through the module. Such adjustment may serve to modify the path length of the air through the module and therefore the dwell time.

If desired, a sensor may be provided with a module to determine the air speed and the inclination of the blades may be modified in dependence upon an output signal of the sensor so as to render the dwell time independent of the air speed.

The treatment of the air during passage through a module may comprise exposure to ultraviolet light. To increase the exposure of the air to ultraviolet light while passing through an ultraviolet module, a UV reflective coating, such as Alanod, may be applied to the inner surface of the housing of the module.

Modules may also deactivate microbes by ozonizing the air, which can additionally serve to remove odours caused by non-microbial compounds. Such ozonizing may be achieved either by UV-C light, or by the creation of an arc discharge.

Though highly effective at deactivating microbes, ozone is potentially harmful if released untreated into the atmosphere. It is possible to place an active carbon filter in the path of the air before it is discharged, but it has been discovered, surprisingly, that exposure to ultraviolet light in the UV-B range is effective in neutralizing ozone, converting it back to oxygen.

For this reason, in some embodiments, UV-B modules may be arranged in series and downstream of ozonizing modules of the purification unit.

If desired, a filter having fibers coated with an anti-microbial agent may be placed in the path of air entering the purification unit. The anti-microbial agent may comprise a quaternary ammonium silane, for example the compound described in US Patent 7,632,797. Any fibrous filters placed in series with the air flow, be it to deactivate microbes or neutralize ozone, need not have a small mesh size and need not therefore create a significant pressure drop. Such filters may serve additionally to trap particulate matter in the air, but the primary function of the purification units of the invention is not to trap particulate matter but to deactivate microbes.

If it is also desired to trap particulate matter, other conventional fibrous filters may be provided in the ventilation system, but as they will not be required to trap microbes, they need not have a small mesh size.

### Brief description of the drawings

The invention will now be described further, by way of example, with reference to the accompanying drawings, in which:
Figures 1a is an end view of a UV-B module,
Figure 1b is a partly cut-away side view of the module of Figure 1a,
Figure 1c is a partly cut-away perspective view of the module in Figures 1a and 1b,
Figure 2 is a partly cut-away perspective view similar to that of Figure 1c of an ozonizing module,
Figure 3 is a perspective view of a diverter having stationary surfaces,
Figure 4 is a perspective view of a diverter having adjustable blades,
Figure 5 shows an ozonizing module mounted in series with a UV-B module,
Figure 6 shows an ozonizing module mounted in series with a reaction module and a UV-B module,
Figure 7 is a perspective view of an arrangement of three modules of differing cross-section connected in parallel with one another,
Figure 8 is a perspective view of two arrangements as shown in Figure 7 mounted in series with one another,
Figure 9 shows an arrangement as shown in Figure 7 that is preceded by a fibrous filter of which the fibers are treated with an anti-microbial composition, and
Figures 10a to 10f are diagrams that demonstrates how modules of different size can be combined to produce purification units of the same capacity yet with different geometries to suit ductwork having different aspect ratios.

### Detailed description of the drawings

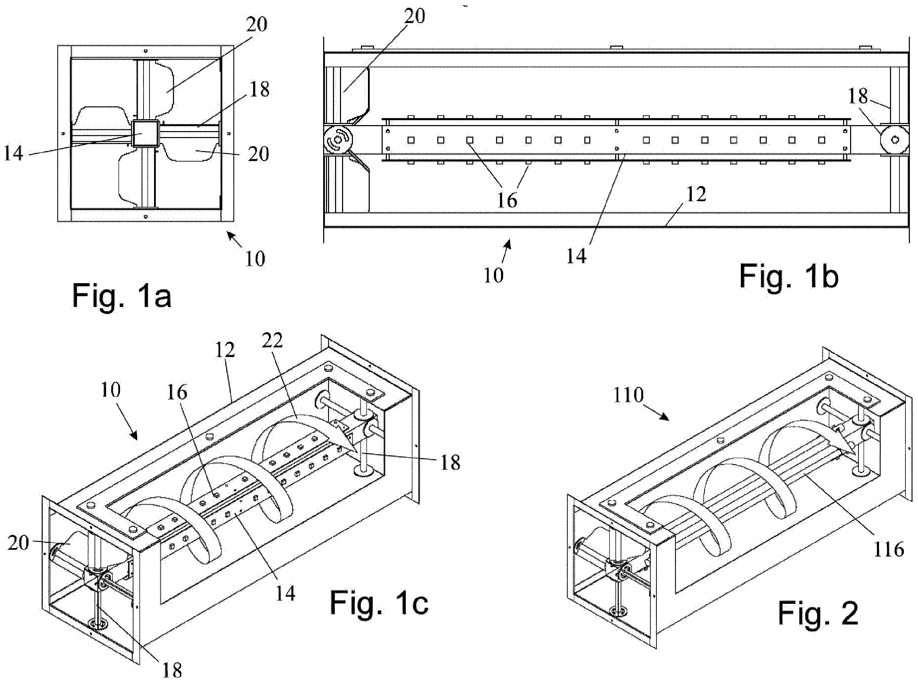

The UV-B module 10 shown in Figures 1a, 1b and 1C comprising a housing 12 in the form of a duct of square cross-section that is open at both ends so that an air stream generated by a fan of a ventilation system may pass through it. The housing 12 is partly cut away in Figures 1b and 1c to reveal a central column 14 on which are mounted a plurality of diodes emitting UV-B light, that is to say light having a wavelength in the range of 240 to 315 nm. The column 14 is held centrally in the housing 12 by means of struts 18 that present minimal obstruction to air flow.

At the intake end of the module 10, shown in Figure 1, blades 20 are mounted on the struts 18 to act as a flow diverter. The inclination of the blades 20 is such that an air stream travelling axially into the module 10 is deflected tangentially by the blades 20 to follow a helical path represented by an arrow 22 in Figure 1c. In this way, the air is made to follow a longer path, so as to increase its dwell time within the module 10.

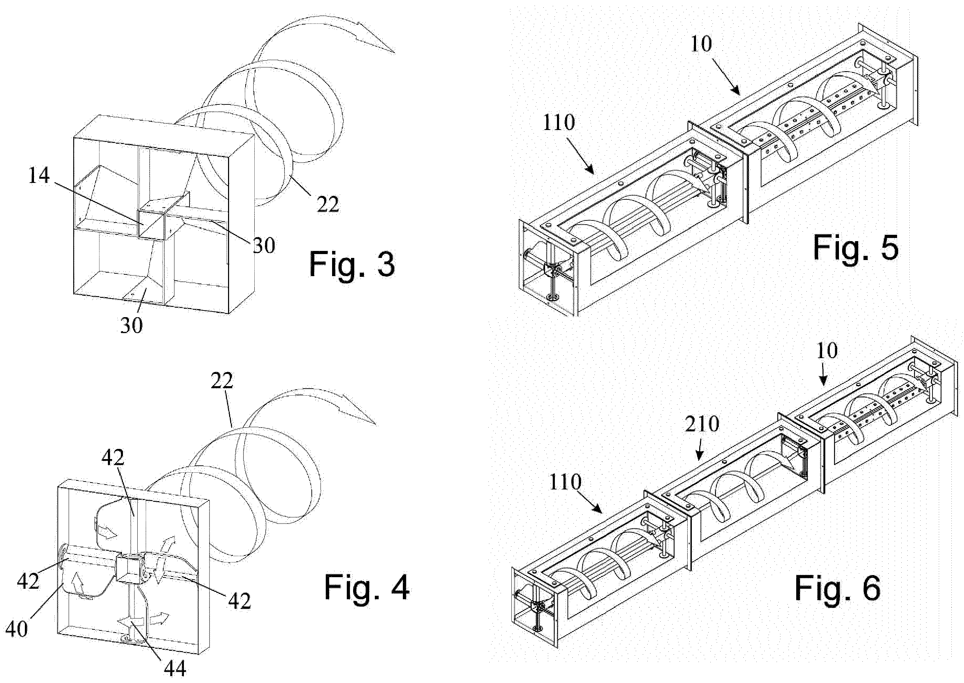

Figure 3 shows an alternative design of flow diverter using stationary deflecting surfaces. In this embodiment, four sheet metal brackets 30 serve both as air deflectors and as struts for supporting the column 14 centrally with the housing 12.

Figure 4 shows a further alternative design of the flow diverter in which the diverter blades 40 are mounted on four independently rotatable shafts 42. In this embodiment, it is possible modify the pitch of the helical path 22 of the air by changing the positions of the blades 40, as represented by the arrows 44 in Figure 4. Altering the pitch changes the path length of the air and its dwell time within the module. To maintain a desired dwell time, it is possible to provide a sensor (not shown) to measure the air speed and to control the inclination of the blades as a function of the measured air speed.

The module 110 shown in Figure 2 has essentially the same construction as the UV-B module 10 save that the UV-B diodes are replaced by tubes 116 emitting UV-C light, that is to say light having a wavelength in the range of 110 to 240 nm. Both UV-B and UV-C light are effective in deactivating microbes, but UV-C also acts to generate ozone, which is a highly effective anti-microbial agent. In place of UV-C tubes 116, it is possible to employ an alternative ozone generator, such as an arc discharge device.

To further increase the exposure of microbes in the air stream to the UV light, the inner surfaces of the housings of both the ozonizing modules and the UV-B modules are designed to reflect the UV-B light.

Though highly effective at deactivating microbes, ozone is potentially harmful and should not be released into the ventilated spaced. Active carbon filters may be used to neutralize ozone, but the present inventors have discovered that exposure to UV-B light is also effective at converting ozone back to oxygen. Thus, in embodiments of the invention, air that has been ozonized is passed through a UV-B module before it is discharged.

Figure 5 shows such an arrangement in which modules are mounted in series with one another so that air passes first through an ozonizing module 110 and then through a UV-B module 10 which serves the dual purpose of deactivating microbes and neutralizing ozone generated in the module 110.

Ozone require some time act and it is possible, as shown in Figure 6, to provide a reaction module 210 between the ozonizing module 110 and the UV-B module 10. The reaction module 210 is constructed in the same manner as the other two modules save that it is essentially empty and contain no sources of UV light or ozone. The function of the reaction module 210 is purely to increase the dwell time during which microbes in the air stream are exposed to ozone.

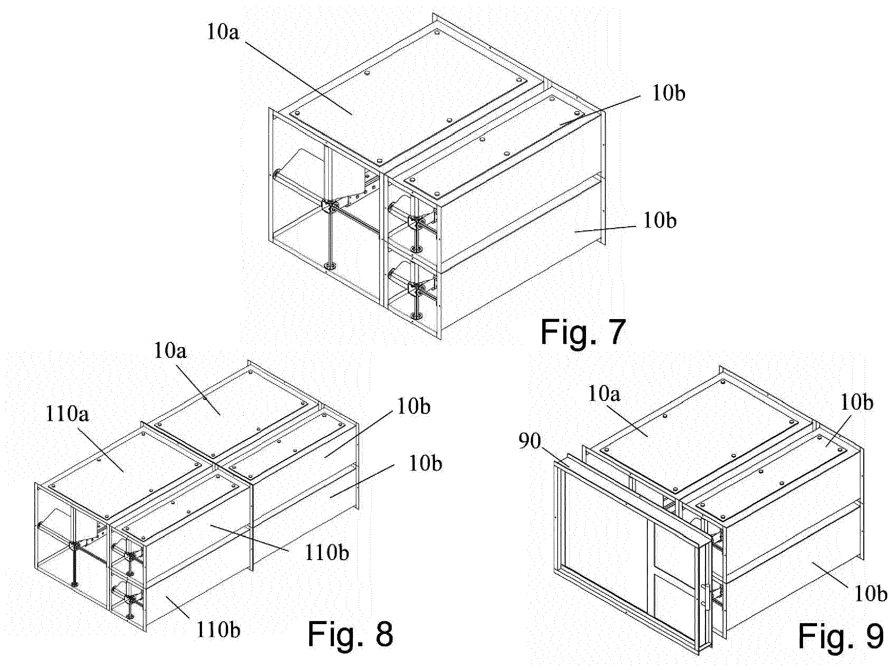

The various modules 10, 110 and 210 of the present invention are manufactured in various different sizes and are designed to the mounted in series with one another, as shown in Figures 5 and 6, in parallel with one another, as shown in Figure 7 or in both series and parallel to one another, as shown in Figure 8. In these figures, modules of different sizes have been allocated reference numerals with different suffixes. In the case of Figure 7, all the modules are UV-B modules, whereas in Figure 8, ozonizing modules are followed by UV-B modules.

Figure 9 shows that a treated filter 90 may if desired be mounted on the upstream side of a purification unit formed of UV-B modules 10a and 10b. The filter 90 has a large mesh size as it is not designed as a trap for particulate matter and therefore does not present significant resistance to air flow. The fibers of the filter are however coated with an anti-microbial agent to assist in purifying the air.

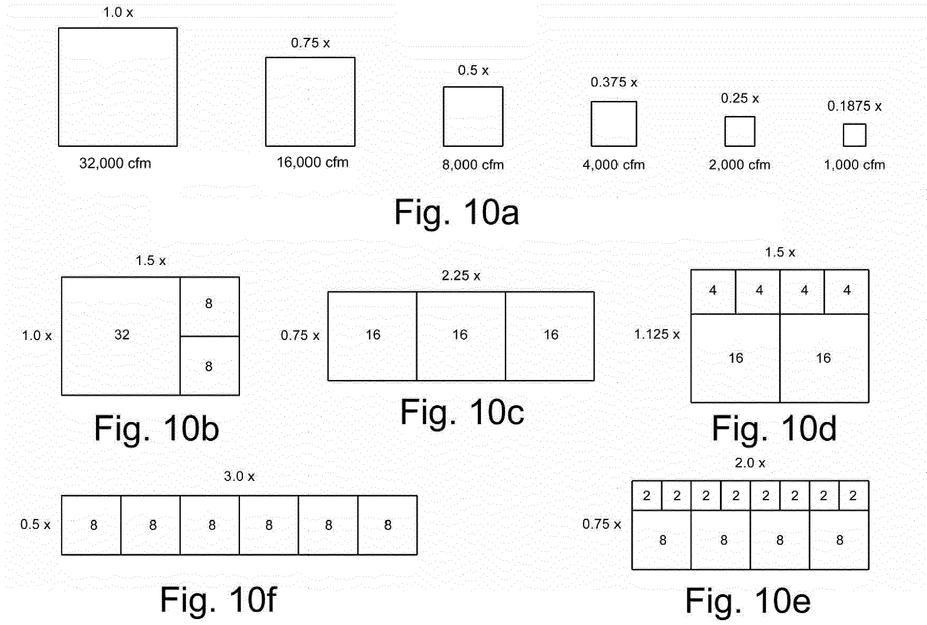

The modules in the present invention are manufactured in specific sizes, shown in Figure 10a, in which the cross-sectional area of each module is given by the formula K.2ⁿ, where K is a constant and n is a positive integer. In the case of the values given in Figure 10a, if the largest module, designed to support a mass flow rate of 32,000 cfm (cubic feet per minute), has a length of 1 unit, then the next size down has half the cross-sectional area to support a mass flow rate of 16,000 cfm. This relationship of halving the cross-sectional area at each stage is repeated until the smallest module (1,000 cfm) is reached, this module having a cross-sectional are that is a thirty second (1/2⁵) of the area of the largest module. Figure 10a also provides an indication of the approximate length of the sides of the modules, whose ratios to one another are powers of the square root of two.

Figure 10b to 10f demonstrate how such modules of different size can be combined in parallel to achieve both a desired total mass flow rate and an overall aspect ratio that is suited to the aspect ratio of the ductwork used in the ventilation system. All five of the combinations shown in Figures 10b to 10f provide a total mass flow rate of 48,000 cfm, yet the aspect ratio may range from 1.5 : 1.125 (Figure 10d) to 6 : 1 (Figure 10f), as well other aspect ratios in between.

## Claims

1. A purification unit for deactivating microbes in an air stream of a ventilation system, wherein the purification unit is composed of modules within each of which flowing air is treated to deactivate microbes, wherein each module has a tubular housing of square cross-section, the cross-sectional areas of different modules are in a ratio of 2ⁿ: 1 to one another, n being a positive integer, and the housings of the modules are mounted in contact and in parallel with one another to form a single purification unit of overall rectangular cross-section.

2. A purification unit as claimed in claim 1, wherein a flow diverter is provided at the intake end of at least some of the modules, to cause air to flow along a helical path through the module.

3. A purification unit as claimed in Claim 2, wherein each diverter comprises inclined surfaces or blades, each serving to deflect tangentially air entering the module axially.

4. A purification unit as claimed in Claim 3, wherein each diverter comprises a rotationally symmetrical arrangement of four surfaces or blades.

5. A purification unit as claimed in Claim 3 or 4, wherein the inclination of the blades is adjustable to vary the pitch of the helical path followed by the air through the module.

6. A purification unit as claimed in Claim 5, wherein a sensor is provided to measure air speed in a module and the inclination of the blades is adjusted in dependence upon an output signal of the sensor in order to render the dwell time independent of the air speed.

7. A purification unit as claimed in any one of claims 1 to 6, comprising at least one module for treating the air passing therethrough by irradiation with UV light.

8. A purification unit as claimed in Claim 7, wherein, in order to increase the exposure of microbes to ultraviolet light while passing through an ultraviolet module, a UV reflective coating is applied to the inner surface of a housing of the module.

9. A purification unit as claimed in any one of claims 1 to 8, comprising at least one module for treating the air passing therethrough by introduction of ozone into the air.

10. A purification unit as claimed in Claim 9, wherein the ozone is generated by UV-C light, or by an arc discharge.

11. A purification unit as claimed in Claim 9 or 19, wherein a module irradiating air with UV-B light is disposed downstream of each module introducing ozone into the air.

12. A purification unit as claimed in any one of Claims 1 to 11, in which a treated filter having fibers coated with an anti-microbial agent is disposed in the path of air entering the purification unit.
